# EUROPEAN PATENT APPLICATION

(11) **EP 2 783 706 A1**
(43) Date of publication of application: **01.10.2014**
(21) Application number: 14161439.6
(22) Date of filing: 25.03.2014
(51) Int. Cl.: A61L 27/36

(54) **Implant material or device**

(30) Priority: 25.03.2013 AT 5004313 U
(71) Applicant: Kaudela, Karl, 2154 Unterstinkenbrunn (AT)
(72) Inventor: Kaudela, Karl, 2154 Unterstinkenbrunn (AT)
(74) Representative: Sonn & Partner Patentanwälte

(57) **Abstract**

The present invention relates to an organic or anorganic implant material or device of human, animal or artificial origin, wherein at least a part of the material or device is covered with a layer comprising a platelet lysate material of human or animal origin and the material or device has an enlarged surface, as well as to a process for the production of such an implant material or device.

## Description

The present invention relates to an organic or anorganic implant material of human, animal or artificial origin with activated surface.

Blood contains plasma, red blood cells, white blood cells and platelets. These platelets are small, discoid cells with a life span of about 7 to 10 days. Inside, platelets contain granules comprising clotting and growth factors. During a healing process after injury, the platelets in the patient's blood are activated and aggregate together, releasing the granules with the growth factors and stimulating the inflammatory cascade and healing process.

Whole blood typically contains 6% platelets, whereas platelet-rich plasma (PRP) has a significantly increased supra-physiological platelet concentration. Although this level can vary depending on the method of extraction and equipment, studies have shown that clinical benefit can be obtained if the PRP used has an increased platelet concentration of 4x greater than normal blood.

Injections of platelet-rich plasma (PRP) have been used and studied since the 1970s. Its use in the treatment of musculoskeletal lesions and injuries has become more popularized over the last several years, but also in cases of doping. In 2010, the World Anti-Doping Code (WADA) then stated that intramuscular injections of platelet derived preparations were not permitted and other routes of administration, e.g. intratendinous or musculotendinous junction injections, required a declaration of use on the doping control form. The latest 2011 code has now deemed all musculoskeletal PRP injections to be considered legal with no notification required.

PRP works by increasing the concentration of platelets, thereby increasing the concentration of growth factors and increasing healing potential. It has been studied and used for the treatment of tendon injuries, chronic wounds, ligamentous injuries, cartilage injuries, muscle injuries, and bone augmentation. The results from in vitro and in vivo studies are promising.

PRP has an advantage over many tissue engineering products in that it is autologous. The main disadvantage of PRP, however, is the necessity of blood puncture of the patient, the need of a centrifugation, the handling as well as the limited amount.

A number of documents have described the possible use of PRP, among others Buhr M., Siekmann W. "Intraartikulare Injektion von thrombozytenangereichertem Plasma zur Behandlung von Knorpelschäden" in Orthopädische Praxis 45, 1, 2009; Hall M. P., et al. "Platelet-rich Plasma: Current Concepts and Application in Sports Medicine", Journal of the American Academy of Orthopaedic Surgeons, Vol. 17, No. 10, Pages 602-608, Oct. 2009; Lopez-Vidriero E., et al. "The Use of Platelet-Rich Plasma in Arthroscopy and Sports Medicine: Optimizing the Healing Environment", Arthroscopy: The Journal of Arthroscopic and Related Surgery, Vol. 26, Issue 2, Pages 269-278, Feb. 2010; Milano G., et al, "The effect of autologous conditioned plasma on the treatment of chondral defects of the knee. An experimental study", International Journal of Immunopathology and Pharmacology, Vol. 24, No.1 Suppl 2, Pages 117-124, Jan-Mar 2011; Redler L. A., et al. "Platelet-rich Plasma Therapy: A systematic Literature Review and Evidence for clinical use", The Physician and Sportsmedicine; Vol. 39, No.1, Pages 42-51, Feb. 2011; Spang J. T., et al. "Platelet concentrate vs. saline in a rat patellar tendon healing model Knee Surgery", Sports Traumatology, Arthroscopy, Vol. 19, No 3; Pages 495-502, 2011 and Sundman E. A., Cole B. J., Fortier L. A. "Growth Factor and Catabolic Cytokine Concentrations are influenced by the Cellular Composition of Platelet-Rich Plasma" The American Journal of Sports Medicine, Vol. 39, No. 10, Pages 2135-2140, Oct. 2011.

Aim of the present invention is to avoid the disadvantages connected with the direct injection of PRP and to provide for an implant material or device with improved healing properties.

The aim is reached according to the present invention in that an organic or anorganic implant material or device of human, animal or artificial origin is provided, at least a part of which material or device is covered with a layer comprising a platelet lysate material of human or animal origin, wherein the material or device has an enlarged surface. Implant materials according to the present invention are understood to be manufactured to replace a missing biological structure, support a damaged biological structure, or enhance an existing biological structure. The term implant is also understood to designate man-made or at least man-processed materials or devices, in contrast to transplants, which are understood to consist of unprocessed biomedical tissue. Thus, the surface of implant materials or devices according to the present invention that contact the body of a patient can be made of a biomedical material, such as titanium, apatite, calcium phosphates or hydroxy apatite or a plastic material like silicone or polyurethanes and/or mixtures thereof. The implant itself can also be an allograft or xenografts and/or at least partially comprise tissue material, like skin or tendons, depending on what is the most functional. The enlarged surface can be achieved for instance by drilling holes into the material or device, by perforating or roughening the surface or by other means well known in the art.

In a preferred embodiment of the present invention, the cover of platelet lysate material extends over the whole surface of the implant material or device, especially preferred is an embodiment where the implant material or device is saturated through and through (i.e. impregnated) with platelet lysate material.

According to a preferred embodiment, the implant material or device of the present invention is manufactured in that an organic or anorganic carrier material for the implant material or device of human, animal or artificial origin is cleaned, provided with an enlarged surface for instance by drilling holes into the material or device, by perforating or roughening the surface or by other means well known in the art, and then at least partially covered with a layer comprising a platelet lysate material of human or animal origin, and then dried, preferably freeze dried.

Prior to the covering, according to a preferred embodiment of the present invention, the implant material or device is cleaned and defatted, for instance by a treatment with supercritical CO₂, followed by chemical washing steps subsequently with H₂O₂, NaOH and Buffer and/or other methods well known in the art. Suitable methods include for instance sterilisation treatment with peroxyacetic acid/ethanol, defatting with chloroform/methanol, drying with acetone, defatting with ultrasonic sound, osmotic treatment with NaCl and H₂O and drying by lyophilisation.

According to a preferred embodiment of the method of the present invention, the at least partial layer comprising a platelet lysate material of human or animal origin is provided for by pouring a suitable solution comprising a platelet lysate material over at least part of the implant material or device and/or dipping the implant material or device into such a solution and/or soaking the implant material or device in such a solution. Preferably, the solution also comprises an antibiotic or combinations of antibiotics, for example one or more members from the group of glykopeptides, like vanomycin or teicoplanin; β-lactame, like penicilline, cephalosporine, monobactame and carbapeneme; polyketides like tetracycline or macrolid-antibiotics; polypeptide-antibiotics like polymyxine, bacitracin and tyrothricin; chinolones like ciprofloxacine or norfloxacine; or sulfonamides.

The thus processed implant material or device can then be implanted into a human or animal body for treatment of human or animal musculoskeletal lesions.

PRP, also called platelet lysate (PL), is a light-yellow liquid that is obtained from blood platelets after freeze/thaw process, which lysate material contains a huge quantity of growth factors. PL or PRP can be prepared from platelets isolated from whole blood distributed in a standard platelet collection bag. There are some differences between protocols, but they share the same core of being frozen at temperatures around -20°C and thawed on the day after. This process is repeated three or four times and the remained supernatant is purified and stored at -20°C.

The source of the PRP or PL used according to the present invention can also be the buffy coat after centrifugation of human blood donations. To release the content of the platelets, several methods well known in the art can be used to create a lysate material. Especially preferred are the methods described in "Preparation of Pooled Human Platelet Lysate (pHPL)" Katharina Schallmoser, Dirk Strunk at http://www.jove.com/details.php?id=1523 or "Development and validation of a production process of platelet lysate for autologous use", Karin Plöderl et al, Platelets, May 2011; 22(3): 204-209.

Covering or impregnating of the implant material or device of the present invention with a homologous allogenic platelet lysate material covers at least part of the surface of the implants with the bioactive agents of the platelet lysate material and leads to an activation of the surface of the implanted material or device. The activated surface enhances the repairing potential of the recipient by activating the healing cascades and leads to new kind of activated enhanced repair and regeneration with no risk of serious side effects. By impregnating the implant material or device according to the present invention it is also safeguarded that the activation remains for a prolonged period of time.

The present invention is now further explained by the following example, to which it should not be limited.

### Preparation of the bone

An implant material was produced starting from a bone of animal origin. First, the bone, a femur of human origin with a length of 18 cm underwent a preparation according to attached Fig. 1 and 2, then any fat present was extracted from the bone by a treatment with supercritical CO₂ according to methods well known in the art, followed by chemical washing steps (subsequently with H₂O₂, NaOH and Buffer).

The bone material thus obtained was then subjected to demineralization of 1 - 2 mm of the bone surface by a 2 hour treatment with 0.5 M EDTA (Ethylenediaminetetraacetic acid), rinsing with purified water and then then placing the treated bone in a buffer.

Then, the bone material underwent vacuum drying with ethanol followed by heating in an oven under vacuum.

The dry bone material was pouched and sterilized with gamma sterilization (25 - 40 KGy) and stored for further use.

### Impregnation with antibiotic and lysate

Frozen lysate (4 tubes, 40 ml each, 160 ml in total) are placed in a fridge (+2/+8 °C) over night before impregnation is scheduled. In case of a frozen core in the morning, the tubes are placed in a waterbath of maximum 37°C (e.g. on a gentle shaking device) for a complete defrost prior to impregnation.

### Impregnation

i. 5 g Vancomycin + 50 ml WFI (water for injection), dissolve and sterile filtration,
ii. shake the defrosted plasma concentrate in the 4 falcon tubes gently and add the liquid lysate concentrate aseptically to the sterile vancomycin-solution, stir/shake gently again (in total 50 + 160 = 210 ml impregnation solution were prepared),
iii. pour the impregnation solution aseptically over the bone prepared as above, preferably the bone is held in a tray of sterilized aluminum foil,
iv. put the tray with the impregnated bone into a Vacuum oven and freeze dryer, vacuum over night and freeze dry for 72 hours.

The impregnated sterile product is then packed into a double pouch under laminar air flow, followed by release based on the reviewed protocols, batch manufacturing record and the packaging record.

## Claims

1. Organic or anorganic implant material or device of human, animal or artificial origin, wherein at least a part of the material or device is covered with a layer comprising a platelet lysate material of human or animal origin, **characterized in that** the material or device has an enlarged surface.

2. Implant material or device according to claim 1, **characterized in that** the cover of platelet lysate material extends over the whole surface of the implant material or device.

3. Implant material or device according to claims 1 or 2, **characterized in that** the implant material or device is impregnated with platelet lysate material.

4. Process for the production of an implant material or device according to any of claims 1 to 3, **characterized in that** an organic or anorganic carrier material for the implant material or device of human, animal or artificial origin is cleaned, provided with an enlarged surface, for instance by drilling holes into the material or device, by perforating or roughening the surface or by other means well known in the art, and then at least partially covered with a layer comprising a platelet lysate material of human or animal origin, and then dried, preferably freeze dried.

5. Process according to claim 4, **characterized in that** the implant material or device is cleaned and defatted by a treatment with supercritical CO₂, followed by chemical washing steps subsequently with H₂O₂, NaOH and Buffer.

6. Process according to claims 4 or 5, **characterized in that** the at least partial layer comprising a platelet lysate material of human or animal origin is provided for by pouring a suitable solution comprising a platelet lysate material over at least part of the implant material or device and/or dipping the implant material or device into such a solution and/or soaking the implant material or device in such a solution.
